# EUROPEAN PATENT SPECIFICATION

(11) **EP 1 071 949 B1**
(45) Date of publication and mention of the grant of the patent: **01.09.2004**
(21) Application number: 99915778.7
(22) Date of filing: 09.04.1999
(51) Int. Cl.: G01N 33/02

(54) **METHOD FOR DETERMINING A GUSHING FACTOR FOR A BEVERAGE**
VERFAHREN ZUM BESTIMMEN EINES ÜBERSCHÄUMFAKTORS FÜR EIN GETRÄNK
PROCEDE POUR DETERMINER UN FACTEUR DE GICLAGE POUR UNE BOISSON

(30) Priority: 17.04.1998 FI 980863
(43) Date of publication of application: 31.01.2001
(73) Proprietor: OY PANIMOLABORATORIO - BRYGGERILABORATORIUM AB, 02151 Espoo (FI)
(72) Inventor: HAIKARA, Auli, FIN-00210 Helsinki (FI); SARLIN, Tuija, FIN-00600 Helsinki (FI); NAKARI-SETÄLÄ, Tiina, FIN-02160 Espoo (FI); PENTTILÄ, Merja, FIN-00210 Helsinki (FI)
(74) Representative: Partio, Erja
(86) International application number: PCT/FI1999/000305
(87) International publication number: WO 1999/054725

(56) References cited:
- DATABASE BIOSIS PREVIEWS DIALOG INFORMATION SERVICES, FILE 5, DIALOG ACCESSION NO. 11609030 BIOSIS NO. 199800390792 KAKUI T. ET AL.: 'Development of monoclonal antibody sandwich-ELISA for determination of beer foam-active proteins' & JOURNAL OF THE AMERICAN SOCIETY OF BREWING CHEMISTS vol. 56(2), 1998, pages 43 - 46
- DATABASE BIOSIS PREVIEWS DIALOG INFORMATION SERVICES, FILE 5, DIALOG ACCESSION NO. 10801781 BIOSIS NO. 199799422926 ISHIBASHI Y(A) ET AL.: 'Application of ELISA to quantitative evaluation of foam-active protein in the malting and brewing processes' & JOURNAL OF THE AMERICAN SOCIETY OF BREWING CHEMISTS vol. 55(1), 1997, pages 20 - 23
- DATABASE BIOSIS PREVIEWS DIALOG INFORMATION SERVICES, FILE 5, DIALOG ACCESION NO. 11783307 BIOSIS NO. 199900029416 MILLS E.N. CLARE(A): 'Immunological study of hydrophobic polypeptides in beer' & JOURNAL OF AGRICULTURAL AND FOOD CHEMISTRY vol. 46(11), 1998, pages 4475 - 4483
- TRENDS IN PLANT SCIENCE JOSEPH G.H. WESSELS: 'Fungal hydrophobins: proteins that fuction at an interface' vol. 1, no. 1, January 1996,, pages 9 - 15, XP000866041
- EUROPEAN BREWERY CONVENTION PROCEEDINGS OF THE 24TH CONGRESS PIA VAAG ET AL.: 'Practical experiences with immunological detection of Fusarium in barley and malt' 1993,, pages 111 - 120, XP000866435

## Description

The present invention relates to a method for determining a gushing factor for a beverage, as defined in the preamble of claim 1.

In beer production, if mould infected barley is used in brewing, the beer produced may be liable to gushing. Gushing is a phenomenon of excessive effervescence of beer, for instance beer may spurt out of a bottle when it is opened. Among the worst factors causing gushing are Fusarium moulds, but gushing activity is also observed in moulds of the Altenaria, Aspergillus, Nigrospora, Penicillium and Stemphylium species. Research on gushing factors produced by moulds has been carried on for decades, but so far it has not been possible to accurately identify and characterise those factors. It has been established that gushing factors are peptides or at least compounds containing peptides. Moreover, they have been found to be of a hydrophobic and acid nature. The gushing factors of most mould fungi are quite rich in cystein. Recent research gives reason to assume that gushing factors are concentrated in the husks of barley.

At present, the gushing propensity of beer is tested by determining the proportion of grains infected with Fusarium moulds in a batch of barley. The batch is rejected if the proportion of infected grains exceeds an allowed limit. This method is laborious and slow to implement and it is not quantitative. The worst weakness of the method is the fact that it cannot be used to determine a real factor that causes gushing, a gushing factor. Therefore, the results may lead to incorrect conclusions.

The object of the present invention is to eliminate the drawbacks mentioned above.

A specific object of the present invention is to disclose a reliable method for determining the gushing propensity of beverages, especially beer, from the raw materials of the beverages, a method that is applicable in quality control. In particular, the object of the method is to determine the amount of a real gushing factor in beer from corn.

The method of the invention is characterised by what is presented in claim 1.

The invention is based on research work carried out to study the factors causing gushing of beer, during which it was established that hydrophobic proteins, hydrophobins, are gushing factors in beer.

Hydrophobins are small hydrophobic proteins produced by moulds and generally containing e.g. 100 ± 25 amino acids. So far, hydrophobins have been isolated from several mould fungi and edible mushrooms. A feature characteristic of hydrophobins is that they have 8 cystein residues which are located in protein in accordance with a certain formula. Hydrophobins occur on the surface of mould mycelium and spores and in secretions in the substratum. Hydrophobins are agents promoting the attachment of mycocelia to the substratum and to each other and they form a protective layer on the surface of the aerial mycelium and spores, protecting them against becoming waterlogged. It has been established that hydrophobins gather at the interface between phases, e.g. on the surface of air bubbles in a culture solution, forming amphipathic films at the interface. Due to their film forming capability, hydrophobins change the surface properties of materials from hydrophobic to hydrophilic and vice versa. Their ability to reduce the surface tension of a water solution is comparable to that of certain synthetic detergents. Hydrophobins are described e.g. in the following articles: Wessels, J.G.H., Hydrophobins: Proteins that Change the Nature of the Fungal Surface, Advances in Microbial Physiology, vol. 38, 1997 Academic Press Limited, pp. 1-45 and Wessels, J.G.H., Fungal hydrophobins: proteins that function at an interface, Trends in Plant Science, vol. 1, s. 9-15. Hydrophobins stabilise bubbles, causing foaming of the culture solution. It has been established that the hydrophobin now isolated from moulds causes gushing of beer.

According to the invention, the amount of hydrophobin in the raw material of a beverage and/or in a beverage, especially in corn, such as barley, malt and/or beer, can be determined by any method applicable for the determination of hydrophobin.

In a preferred embodiment, hydrophobin is determined immunologically by using an immunological reaction between a hydrophobin antigen and an antibody. The immunological method may be an immunoradiometric, immunoenzymetric, immunofluorometric or immunoluminometric method. The antibodies specific to hydrophobin proteins which are needed in the determination are produced by conventional methods of producing antibodies.

Hydrophobin proteins can be isolated from mould strains having a gushing activity, especially from Fusarium strains. Usable strains are e.g. Gibberella avenacea (F.avenaceum), F.culmorum, F.poae, Gibberella zeae (F.graminearum), Nigrospora sp. and T.reesei. Hydrophobins can be isolated from mould mycelium and/or culture solutions by conventional methods e.g. by extracting, bubbling and/or cold drying.

Hydrophobin can be isolated from mycelium by a three-phase extraction method and/or from a culture solution by bubbling the solution, causing concentration of hydrophobin in the foam produced, and/or by deep-freezing the culture solution, causing sedimentation of hydrophobin, which can then be separated by centrifuging the solution after it has been melted.

In a preferred embodiment of the method for determination of hydrophobin, the ELISA (Enzyme Linked ImmunoSorbent Assay) method is used. The colour or fluorescence produced in the enzymatic reaction in the method indicates the presence and quantity of hydrophobin. The ELISA method, the preparation and purification of antibodies and the enzymes, conjugates and substrates used in it are described e.g. in the following article. Vaag, P., Enzyme-Linked ImmunoSorbent Assay (ELISA) in the Beverage Industries: Principles and Practice, Analysis of Non-Alcoholic Beverages (Modern Methods of Plant Analysis, new series vol. 8), Eds. Liskens, H.H. and Jackson, J.F., Springer-Verlag, Berlin 1988, pp. 1-29.

In the method of the invention, other corresponding immunological procedures can also be used, such as the EBStrALISA (Enzyme Biotin Streptavidin Linked ImmunoSorbent Assay) method. The EBStrALISA method is described e.g. in the article Vaag, P., Immunological detection of Fusarium in barley and malt, Proc. Eur. Brew. Conv. Lisbon 1991, pp. 553-560.

In a second preferred embodiment of the method, hydrophobin is determined immunochromatographically using a test strip based on immunochromatography. The colour produced in the immunological reaction in the method indicates the presence and quantity of hydrophobin.

The test strip used may comprise e.g. a chromatographic membrane provided with moving marking particles and two stationary hydrophobin antibody areas. The sample is absorbed into the test strip membrane, where it reacts with the marking particles and drifts into the antibody areas on the membrane. If the sample contains hydrophobin, coloured test and control lines will appear in the antibody areas as a result of an immunological reaction. If the sample does not contain any hydrophobin, only a control line will appear.

The invention makes it possible to replace the semi-quantitative detection method based on indication of Fusarium fungus as used in industrial quality control with a new reliable precision method when a primary gushing factor is to be determined from corn. By selecting the corn to be used, especially barley, by the new method, it will be possible to reduce the need for precautions, which have proved to be expensive and inefficient, especially in risk years when corn quality is low and beer produced from corn shows a strong tendency to gushing.

Further, the new immunological detection method provides a faster and simpler and therefore cheaper method for determining the gushing tendency of barley.

In the following, the invention will be described in detail by means of a few examples.

### Example 1. Isolation and indication of hydrophobins

Hydrophobin was isolated from mould strains having a gushing activity: Gibberella avenacea (F.avenaceum) (VTT-D-80141), F.culmorum (VTT-D-80148), F.poae (VTT-D-82182), Gibberella zeae (F.graminearum) (VTT-D-95470), Nigrospora sp. (VTT-D-79122) and T.reesei (VTT-D-74075).

Moulds were cultivated on three different substrates: on agar, in culture solution and in barley. Hydrophobin was isolated by a three-phase extraction method and by bubbling the culture solution. The molecular distribution of the proteins contained in the sample was established via gel electrophoresis. The hydrophobin produced by the T.reesei strain is of a size about 7.5 kDa. The hydrophobins produced by the Nigrospora sp). (mycelium extraction) and F.poae (bubbling of culture solution) strains were of about the same size. The hydrophobin extracted from the mycelium of the Gibberella zeae strain was of a size about 20 kDa.

For the indication of hydrophobins in a sample, the immunological ELISA test was used. The antibody used in the test was prepared using the following immunising protocol. A rabbit was immunised using a hydrophobin produced by T.reesei mould, which was mixed with Freund's adjuvant. Injections were given 4 times during three months. Titre development was monitored by determining the antibody content of the blood twice using the ELISA method. At the end of the immunising period, the rabbit's blood was collected and the serum was separated from the blood. The reactivity of the serum was tested by determining the weakest serum dilution that still gives a response in the ELISA method. The dilution was over 1/100 000.

In the test, a hydrophobin sample was pipetted into the pits on a microtitre plate, with the result that the proteins in the sample became attached to the walls of the pits. Hydrophobin antibody was then added, and it recognised the hydrophobin protein on the walls and attached to it. After this, a conjugate was added, which in turn attached to the antibody. The conjugate contained an enzyme which produced colouring of the substrate pipetted into the pits. The intensity of the colour of the substrate was directly proportional to the hydrophobin content of the sample.

The highest responses were obtained from hydrophobin samples of F.poae, Nigrospora sp. and T.reesei.

### Example 2. Determination of hydrophobin from a barley sample

Barley was subjected to contamination with the Fusarium strains and T.reesei strain of example 1 for two weeks. A barley extract was prepared by mixing 50 g of barley/100 ml of water for 1 minute. The barley-water mixture was centrifuged for 15 minutes at a velocity of 4000 g. The supernatant extract was collected and the hydrophobin contained in it was determined by the direct ELISA method using an antibody prepared against T.reesei hydrophobin.

On microtitre plates, barley extract diluted to 1/10 and 1/100 was pipetted in an amount of 150 µl/pit. The dilutions were prepared in a sodium phosphate buffer (10 mM of sodium phosphate pH 7.3, 150 mM of sodium chloride = PBS solution). The plate was kept in a refrigerator overnight. On the day, PBS solution containing 0.1 % bovine serum albumen and 0.05 % Tween 20 solution (=BSA/PBS solution) was pipetted onto the plate in an amount of 100 µl/pit. The plate was held at a low temperature overnight, whereupon it was washed with a PBS solution containing 0.01 % Tween 20 solution added to it (PBST solution). From hydrophobin antibody, a 1/100 dilution of was made in the BSA/PBS solution. Of this antibody dilution, 100 µl/pit was pipetted onto the plate and the plate was incubated for 2h at +37°C. The plate was washed with PBST solution. Next, 100 µl/pit of conjugate: goat's anti-rabbit lgG alkaline phosphatase diluted to 1/1000 in BSA/PBS solution, was pipetted onto the plate. The plate was incubated for 2h at +37°C and washed with PBST solution. Finally, 100 µl/pit of substrate: 1 tablet of p-nitrophenyl phosphate/5 ml of diethylene amine+MgCl₂ buffer, was pipetted onto the plate. The plate was incubated in a shaker for 30 min at room temperature. The absorbance was measured at wavelength 405 nm using a Multiscan photometer. The intensity of colour is directly proportional to the amount of hydrophobin contained in the sample. Table 1 presents the absorbance values of the barley samples.

**Table 1.**

| Sample | Sample 1/10 | dilution 1/100 |
|---|---|---|
| Control | 0.551 | 0.388 |
| VTT-D-80141 | 1.823 | 1.360 |
| VTT-D-80148 | 0.452 | 0.374 |
| VTT-D-82182 | 1.088 | 0.898 |
| VTT-D-95470 | 0.664 | 0.634 |
| VTT-D-74075 | 0.863 | 1.914 |

The highest hydrophobin contents were determined from barley samples contaminated with Gibberella avenacea, F.poae and T.reesei mould strains.

The method is also well applicable for use with malt samples.

### Example 3. Beer gushing tests

A beer gushing test was performed by adding 0.5 - 1 ml of hydrophobin sample into a beer bottle. The bottle was shaken for 3 days at room temperature, whereupon it was opened and the amount of beer gushing out was determined from the change in the weight of the bottle.

The hydrophobin samples of T.reesei (mycelium extraction) and F.poae (bubbling of culture solution) produced particularly intensive gushing. The gushing in the case of the former sample was even more than 50 %, and in the case of the latter sample 30 - 45 %. The hydrophobin sample of Nigrospora sp (mycelium extraction) caused repeated gushing of 1 - 10 %. The hydrophobin samples of the other moulds tested also caused gushing of beer in varying degrees.

On the basis of the tests carried out, it can be stated that hydrophobin proteins cause gushing of beer.

### Example 4. Hydrophobin sequencing

The hydrophobin samples in Example 1 of Fusarium poae (bubbling of culture solution) and Nigrospora sp (mycelium extraction) causing gushing of beer were fractionated using the technique of reversed-phase HPLC chromatography (High Performance Liquid Chromatography) (apparatus: Äkta Explorer, Pharmacia Biotech; column: C4, Vydac). The operating buffers used were 0.1 % trifluoroacetic acid (TFA) in water (A) and 0.1 % TFA in acetonitrile (B) . The gradient developed so that operation was started with buffer A and the proportion of buffer B increased in the course of the operation so that finally the operating solution consisted of buffer B only. Proteins like hydrophobin were eluted when the concentration of buffer B was about 50 %. From the fractions obtained, a sodium dodecylsulphate-polyacrylamide gel electrophoresis (SDS-PAGE) was made by means of a Phast apparatus (Pharmacia) using 20 % Phast gels. On fractions containing hydrophobin-sized proteins, an N-terminal sequence analysis was made. A characteristic of the order of aminoacids in hydrophobins is that there are eight cystein residues located in the protein according to the following formula (Wessels, J.G.H. (1996) Fungal hydrophobins: proteins that function at an interface, Trends in plant science. 1:9-15):

X₂₋₃₈-C-X₅₋₉-C-C-X₁₁₋₃₉-C-X₈₋₂₃-C-X₅₋₉-C-C-X₆₋₁₈-C-X₂₋₁₃

X = aminoacid other than cystein
C = cystein Cys.

For the sample fractions, the following partial N-terminal sequences were obtained:
F.poae: 
TPPGYGGGGGSGSNFDA C PGALYSQTQ CC SAGVG-
DIVDV...
i.e. X₁₈-C-X₉-C-C ....
Nigrospora sp.: 
TNDQPATGFVA C ANNGVLFSAPN CC ATDVLGLADLD C
TTPPKVPTSPXDFQ...
i.e. X₁₁-C-X₁₁-C-C-X₁₁-C ...

T = Thr, P = Pro, G = Gly, Y = Tyr, S = Ser, N = Asn, F = Phe, D = Asp, A = Ala, L = Leu, Q = Gln, V = Val, I = Ile, K = Lys.

The sequences for the samples are well in agreement with the sequential formula for hydrophobins. When a sample fraction of Nigrospora sp. was added to beer and a gushing test was performed, half of the beer gushed out. Fractions that did not contain any hydrophobin-sized proteins did not produce any gushing.

The invention is not restricted to the examples of its embodiments described above, but many variations are possible within the scope of the inventive idea defined in the claims.

## Claims

1. Method for determining a gushing factor for a beverage, **characterised in that** the quantity of hydrophobin is determined from the raw material of the beverage and/or from the beverage.

2. Method as defined in claim 1, **characterised in that** the gushing factor in beer is determined by determining the quantity of hydrophobin from the corn, malt and/or beer.

3. Method as defined in claim 1 or 2, **characterised in that** hydrophobin is determined immunologically using an immunological reaction between a hydrophobin antigen and an antibody.

4. Method as defined in any one of claims 1 - 3, **characterised in that** hydrophobin is determined by the ELISA method, the EBStrALISA method and/or immunochromatographically.

## Patentansprüche

1. Verfahren zum Bestimmen eines Überschäumfaktors für ein Getränk, **dadurch gekennzeichnet, dass** die Menge an Hydrophobin aus dem Ausgangsmaterial des Getränks und/oder aus dem Getränk bestimmt wird.

2. Verfahren wie in Anspruch 1 definiert, **dadurch gekennzeichnet, dass** der Überschäumfaktor in Bier durch Bestimmen der Menge an Hydrophobin aus dem Getreide, Malz und/oder Bier bestimmt wird.

3. Verfahren wie in Anspruch 1 oder 2 definiert, **dadurch gekennzeichnet, dass** das Hydrophobin immunologisch bestimmt wird, wobei eine immunologische Reaktion zwischen einem Hydrophobinantigen und einem Antikörper verwendet wird.

4. Verfahren wie in einem der Ansprüche 1-3 definiert, **dadurch gekennzeichnet, dass** das Hydrophobin durch das ELISA-Verfahren, das EBStrALISA-Verfahren und/oder immunchromatografisch bestimmt wird.

## Revendications

1. Procédé pour déterminer un facteur de giclage pour une boisson, **caractérisé en ce que** la quantité d'hydrophobine est déterminée à partir de la matière première de la boisson et/ou à partir de la boisson.

2. Procédé selon la revendication 1, **caractérisé en ce que** le facteur de giclage dans la bière est déterminé en déterminant la quantité d'hydrophobine à partir du maïs, du malt et/ou de la bière.

3. Procédé selon la revendication 1 ou 2, **caractérisé en ce que** l'hydrophobine est déterminée immunologiquement en utilisant une réaction immunologique entre un antigène de l'hydrophobine et un anticorps.

4. Procédé selon l'une quelconque des revendications 1 à 3, **caractérisé en ce que** l'hydrophobine est déterminée par la méthode ELISA, par la méthode EBStrALISA et/ou de façon immunochromatographique.
